# EUROPEAN PATENT APPLICATION

(11) **EP 2 077 110 A1**
(43) Date of publication of application: **08.07.2009**
(21) Application number: 07828729.9
(22) Date of filing: 28.09.2007
(51) Int. Cl.: A61K 31/198, A23K 1/16, A23L 1/305, A61K 8/44, A61K 47/32, A61P 9/00, A61P 17/16, A61P 21/00, A61P 25/28, A61Q 19/02

(54) **GLUTAMINE-CONTAINING COMPOSITION FOR INCREASING BLOOD FLOW**

(30) Priority: 29.09.2006 JP 2006266544
(71) Applicant: Ajinomoto Co., Inc., Tokyo 104-8315 (JP)
(72) Inventor: OHTA, Fumio, Kawasaki-shi, Kanagawa 210-8681 (JP); TAKAGI, Tomo, Kawasaki-shi, Kanagawa 210-8681 (JP); SATO, Hiroyuki, Kawasaki-shi, Kanagawa 210-8681 (JP)
(74) Representative: Nicholls, Kathryn Margaret
(86) International application number: PCT/JP2007/068988
(87) International publication number: WO 2008/038771

(57) **Abstract**

The present invention provides a glutamine-containing composition for increasing blood flow, which contains glutamine in an amount ranging from 25 mg/kg body weight to 150 mg/kg body weight per a dose. According to the composition, the blood flow in the capillary vessels can be increased efficiently, while inhibiting side effects such as low blood pressure. The present invention also provides a food or feed containing the composition for increasing blood flow.

## Description

### [Technical Field]

The present invention relates to glutamine-containing compositions for increasing blood flow. Specifically, the present invention relates glutamine-containing compositions for increasing blood flow in the capillary vessels of a subject. The glutamine-containing compositions of the present invention are formulated as pharmaceutical products, foods, beverages, animal feed and the like.

### [Background Art]

Blood enables the transport of oxygen, nutrients, chemical messenger such as hormones, waste products and immune cells. Namely, substances are taken up by the blood from the lungs, digestive tract and endocrine organs, and then they are propelled by the heartbeats and transported to the entire body via the capillary vessels. Furthermore, waste products and the like are picked up by the blood through the capillary vessels of the entire body and finally excreted from the organs, such as the lungs and kidneys. Blood also functions to retain heat in the body by circulating blood that is always kept at a constant body temperature. Thus, blood flow in the capillary vessels is important for maintaining the vital functions, but it can weaken when stresses occur, such as disease including infectious disease, injury, fatigue, aging, and sudden environmental changes. For example, it is possible that decreased blood flow in the brain may be related to cerebrovascular dementia and Alzheimer-type dementia, wherein the decreased blood flow causes deterioration of the vital functions (NPL 1). In addition, it is reported that metabolism of the livers is influenced by the amount of blood flow (NPL 2). Accordingly, it is important to actively increases blood flow under such conditions so that heat retention in the body is increased and substances which actively enhance the vital functions are effectively transported by the increased blood flow.

Agents which have been used to increase blood flow include calcium antagonists, cellular respiration activators, anticonvulsants, *in vivo* enzymes, and the like.

Some of those conventional blood flow enhancer have been developed for the purpose of reducing blood pressure. Such substances are designed to affect the blood vessels in every part of the body so that there is a possibility of not only increasing the blood flow in the capillary vessels, but also affecting the systemic blood pressure. Some of the *in vivo* enzymes, such as kallikrein, have another effect, for example, a pain producing effect. Therefore, it is strongly desirable to develop a method which is effective to increase blood flow and that is safe and easy to use.

It has been previously reported that arginine induces vasodilation. As its mode of action, it has been reported that vascular endothelial cells which contain nitric oxide synthases synthesize nitric oxide from arginine, which causes vasodilation. Externally administered arginine promptly synthesizes nitric oxide, and this action also produces vasodilation. However, dietary arginine does not usually have the same effect (NPL 3).

Although the effects of arginine when ingested have been widely reported, it has been conventionally thought that vasodilation caused by the action of arginine results in an increase in blood flow as well as a lowering of blood pressure. For example, it has been reported, when 30g or 6g of arginine was intravenously administered to humans having a body weight of 78kg on average, the 30g of arginine lowered blood pressure and vascular resistance, while the 6g of arginine did not cause either effect (NPL 4). In addition, other methods for increasing blood flow using arginine has been reported. In every such case, however, the dosage of arginine is very high, or the arginine must be combined with other compositions which alone can act to increase blood flow, such as polyphenols (NPL 5, PL 1 and PL 2). Therefore, increasing blood flow using solely arginine without lowering blood pressure has never been reported. Under such circumstances, the inventors of the present application have intensively studied the blood flow and blood pressure when arginine was administered, and finally accomplished the invention which is an administration method for increasing blood flow without lowering blood pressure.

### [Summary of Invention]

### [Technical Problem]

An object of the present invention is to provide a composition which can efficiently increase blood flow in the capillary vessels.

### [Solution to Problem]

The inventors carefully observed the changes in blood flow in the capillary vessels after administration of glutamine, and found that administration of glutamine in a certain dosage range increased blood flow in the capillary vessels, particularly the capillary vessels in the livers and the brain. The present invention has been thus accomplished based on this findings.

Namely, the present invention provides a glutamine-containing composition for increasing blood flow, which contains glutamine in an amount ranging from 25 mg/kg body weight to 150 mg/kg body weight per a dose.

Also, the present invention provides a composition for increasing blood flow, which consists of glutamine in an amount ranging from 25 mg/kg body weight to 150 mg/kg body weight per a dose.

The present invention also provides a pharmaceutical composition for preventing, alleviating or treating dementia, cold constitution, shoulder stiffness, skin muddiness, impaired liver function, or lowered immunity, comprising the composition for increasing blood flow as described above.

Furthermore, the present invention provides a food for increasing blood flow, which contains glutamine in an amount ranging from 25 mg/kg body weight to 150 mg/kg body weight per a dose.

The present invention also provides feed comprising the composition for increasing blood flow as described above.

### [Advantageous Effects of Invention]

According to the present invention, the blood flow in the capillary vessels can be increased effectively while inhibiting side effects such as low systemic blood pressure. In particular, the invention makes it possible to increase blood flow in the capillary vessels of the livers and the brain.

### [Description of Embodiments]

The glutamine which can be used in the present invention includes L-glutamine, L-glutamine halides, preferably L-glutamine chlorides, L-glutamine derivatives which can be promptly metabolized to L-glutamine *in vivo*. The L-glutamine derivatives may be in the form of esters, amides, peptides, proteins, pyrrolidone carboxylic acids and the like, and peptides containing L-glutamine as a constituent amino acid, for example. In particular, L-glutamine is preferable in the present invention.

The content of glutamine in the compositions for increasing blood flow of the present invention is ranging from 25mg/kg body weight to 150mg/kg body weight per a dose, and preferably 50 to 150mg/kg body weight per a dose.

The amount of glutamine in the composition for increasing blood flow of the present invention may preferably correspond to a dose capable of increasing the concentration of free glutamine in the blood plasma by 15 to 180 mg, preferably 25 to 180 mg, more preferably 50 to 180 mg, and yet more preferably 50 to 150 mg, per milliliter of blood plasma. A single dose of the composition of the present invention is consistent with these increases. The concentration of the free glutamine in the blood plasma can be determined by taking a blood plasma 10 minutes after the administration of glutamine, and using an amino acid analyzer (the amino acid analyzer L8500 made by Hitachi, Ltd.).

In the present invention, when the blood flow increases by 20% or more relative to the blood flow before administrating the composition of the present invention, it is regarded that the blood flow is increased. This is because the control can sometimes vary as much as 19%.

In the present invention, the composition may further comprise at least one selected from the group consisting of pyrrolidone carboxylic acid, and salts and derivatives thereof. This is because pyrrolidone carboxylic acid, salts or derivatives thereof, helps arginine to be introduced into cells (JP 2004-18483 A). This results in the enhancement in the production of nitric oxide in the endothelial cell, which may further increase blood flow by the action of glutamine according to the present invention. In the present invention, glutamine may be used in combination with glutamic acid instead of pyrrolidone carboxylic acid. This is because pyrrolidone carboxylic acid can be produced from glutamic acid in an aqueous solution (Journal of Agricultural and Food Chemistry 2000; 48: 6003-6010).

The pyrrolidone carboxylic acid, salts or derivatives thereof which can be used in the present invention are preferably in L-form. Such pyrrolidone carboxylic acids may form salts with alkali metals such as sodium and potassium, alkaline earth metals such as calcium, basic amino acids such as arginine and lysine, or amines such as triethanolamine. The pyrrolidone carboxylic acids may also form derivatives with alcohols and may be in the form of acid anhydrides, peptides, proteins and the like. Particularly, sodium salt is preferable.

The glutamic acid which can be used in the present invention includes L-glutamic acid and peptides containing L-glutamic acid as the constituent amino acid. The glutamic acids may form salts with alkali metals such as sodium and potassium, alkaline earth metals such as calcium, basic amino acids such as arginine and lysine, or amines such as triethanolamine. The glutamic acid may also form derivatives with alcohols and may be in the form of acid anhydrides, peptides such as wheat gluten hydrolysate, proteins such as wheat gluten. Particularly, L-glutamic acid is preferable.

In the present invention, the amino acids selected from the group consisting of the pyrrolidone carboxylic acid, and salts and derivatives thereof can be administered in combination with glutamine. The dosage of such amino acids is not particularly limited, but may be preferably 6.25 mg/kg body weight to 600 mg/kg body weight, and more preferably 25 mg/kg body weight to 300 mg/kg body weight in each single dose.

The ratio by mass of glutamine to such amino acid(s) in the composition for increasing blood flow of the present invention may be from 1:4 to 4:1, and more preferably from 1:2 to 2:1. It is preferable that the composition of the present invention contain glutamine and the amino acid(s) in the above-stated ratio, because the volume of blood flow significantly increase.

The composition for increasing blood flow of the present invention can preferably be used for preventing, alleviating or treating cerebrovascular dementia, Alzheimer-type dementia, shoulder stiffness, cold constitution, skin muddiness, muscle fatigue, impaired liver function, lowered immunity and the like. The increased blood flow can prevent, alleviate or cure dementia, cold constitution, shoulder stiffness, skin muddiness or muscle fatigue, as reported in Biomedical Gerontology 2001: 25(2); 83-88, Bulletin of Yamanashi Medical College 1999: 16; 15, Nitto Denko Technical Report 1992: 30, 1, JP 2002-255827 A, and JP 2001-316256 A. In addition, it is generally known that administration of glutamine does not induce lowering of blood pressure (Journal of Surgical Research 1992: 52; 499). The composition of the present invention can effectively increase blood flow especially in the capillary vessels, particularly those in the skin, spleen, livers, heart and brain, while inhibiting the lowering of the systemic blood pressure, and therefore, more effectively distribute blood flow in the body.

The composition of the present invention can be prepared into not only the form of pharmaceutical compositions, but also foods, health foods, dietary supplements, nutritional compositions, feed for livestock or domestic animals or the like. These food compositions should indicate on their packaging that they are useful for increasing blood flow. The dosage generally varies depending on body weight, health conditions, and the like of a subject ingesting the food compositions. When administering to adults, 4.5 to 27g, more preferably 9 to 27g of glutamine per day is a preferable dose. The compositions may be packed in any product form so that the unit of product contains 1 to 27g of glutamine, for example, 1g, 2g, 3g, 4g, or 4.5 to 27g. When the glutamine amount per unit is 1g, 2g, 3g, 4g or the like, a plurality of units may be administered daily.

To form the pharmaceutical composition, pharmaceutically acceptable carriers or diluents, for example, cellulose derivatives (carboxymethyl cellulose, ethyl cellulose and the like), starches (potato starch, corn starch and the like), sugars (lactose, sucrose and the like), vegetable oils (peanut oil, corn oil, sesame oil and the like), polyethylene glycol, alginic acid, gelatin, talc, and the like may be added to prepare a variety of dosage forms, such as orally administered preparations, e.g., tablets, powders, pills, granules, capsules, syrups and the like; injections, e.g., subcutaneous injections, intravenous injections, intramuscular injections, epidural injections, subarachnoidal injections, and the like; external preparations, e.g., nasally administered formulations, transdermally administered formulations, ointments and the like; suppositories such as rectal suppositories, vaginal suppositories and the like; intravenous fluids and the like.

The pharmaceutical composition of the present invention may further comprise other active ingredients typically used as pharmaceuticals, including agents for the central or peripheral nervous system, agents for the circulatory organs, hormone preparations, antihormone preparations, vitamin preparations, revitalizers, detoxicating agents, antitumor agents, antiallergic agents, crude drugs, Chinese herbal medicines, chemotherapeutic agents, biological preparations, diagnostic agents, and the like.

The pharmaceutical composition of the present invention may be orally or parenterally administered via, for example, the rectal route or the intravenous route.

With respect to food compositions, nonconventional food forms including supplements and the like are included, as well as conventional food forms. Those food compositions of the present invention can be prepared in the conventional manners by the addition of appropriate additives. Examples of such additives include any ingredients typically used in health food products, such as fruit juice to adjust and enhance the taste, dextrin, cyclic oligosaccharides, sugars (fructose and glucose syrup, sucrose), acidifiers, flavoring agents, green tea powder, and fats and oils, emulsifiers to improve the texture, collagen, whole milk powder, thickening polysaccharides, agar (employed for jelly-like beverage) and the like.

The food compositions of the present invention may further comprise amino acids, vitamins, eggshell calcium, calcium pantothenate and other minerals, royal jelly, propolis, honey, dietary fibers, agaricus, chitin, chitosan, capsaicin, polyphenols, carotenoid, fatty acids, mucopolysaccharides, coenzymes, antioxidants and the like to prepare health food products.

The composition for increasing blood flow of the present invention may also be formed into feed for mammals such as swine, bovines, sheep, canines, felines, mice, rats and monkeys and the like. For example, a solid or liquid additive for in feed can be prepared in accordance with the methods conventionally known in the art.

The product form of the composition for increasing blood flow of the present invention are not particularly limited, and include product forms which are typically used for delivery of amino acids. For oral administration, examples include powders, granules, tablets, liquids (e.g., beverages and jelly-like beverages) and sweets (e.g., chocolates), wherein a suitable excipient(s) are used, or the mixture of one or two kinds of the above amino acids. For intravenous administration, examples include infusion solutions and aqueous solutions, each containing one or two kinds of the above-mentioned amino acids, and amino acid powders that can be added before administration.

The blood flow in the capillary vessels is an important index indicating the effects of the present invention. Capillary blood flow can be measured using a laser Doppler blood flowmeter (FLO-N1, made by OMEGAWAVE, Inc.) or microspheres (Dye-Trak VII+, made by Triton Technology Ltd.).

### [Examples]

The present invention will now be further illustrated with reference to the following Examples.

Example (Determination of changes in blood flow in various organs after oral administration of glutamine to rats)

### (1) Summary of Experiment

(a) Glutamine was given to rats and then the changes in blood flow in the capillary vessels of their various organs were determined.
(b) Male SD rats having a body weight of about 400 g were subjected to the experiment.
(c) The rats were anesthetized by pentobarbital, and catheters were placed in the left ventricle and the right femur of each rat. After causing the rats to recover from the anesthesia, glutamine was injected into the stomach of each rat through a feeding needle in such an amount that 50 mg/kg body weight of amino acid was administered. Through the catheter located from the carotid artery to the left ventricle, yellow and red microspheres were each injected before and after the administration of the amino acid solution. The amounts of microspheres which were distributed from the heart to the capillary vessels of each organ of the entire body were determined to make comparisons of the blood flow of each organ before and after the administration of glutamine. For the control group, distilled water was administered.
(d) It was observed that glutamine increased the blood flows in the abdominal skin, spleen, livers, heart and brain more than in other organs.
(e) From the above results, a significant increase in blood flow in the capillary vessels of the abdominal skin, spleen, livers, heart and brain is shown in the rats to which 50 mg/kg of glutamine was administered. Therefore, when glutamine is administered according to the present invention, the effect is considered to be significantly noticeable in the abdominal skin, spleen, livers, heart and brain.

### (2) Details of Experiment

(a) Composition administered in each group: shown in Table 1.

**[Table 1]**

| | | |
|---|---|---|
| Administration Group | Composition | Dose of Total Amino Acids |
| | | Dose of Glutamine |
| Control group | distilled water | Total amino acids: 0 mg/kg |
| | | Glutamine 0 mg/kg |
| Glutamine- | | Total amino acids: 50 mg/kg |
| administered group | | Glutamine: 50 mg/kg |

(b) Operation: Catheters were inserted into the carotid arteries and the femoral arteries of the rats under anesthesia, and through those catheters the microspheres were injected and blood samples were taken out.
(c) Determination of blood flow: Immediately before and after the administration of glutamine, which lasted 30 minutes, yellow and red microspheres were each injected into the left ventricle of each rat through the catheter. With a sufficient interval placed after the second injection of the microspheres, the rats were euthanized, and then the muscles, alimentary canals, livers, kidneys, spleen, heart, brain, fat tissues and abdominal skin were extracted. The microspheres included in each organ were collected and the change ratio of blood flow calculated from the amounts of microspheres was determined for each organ.
(d) Experimental results: The change in blood flow observed in the glutamine administration test is shown in Table 2.

**[Table 2]**

| Change in blood flow after administration of amino acid (calculated on a basis of 100% blood flow before administration) | | |
|---|---|---|
| | Control Group (%) | Glutamine-Administered Group (%) |
| Gastrocnemius | 94 | 105 |
| Soleus | 91 | 146 |
| Abdominal skin* | 94 | 146 |
| Dorsal skin | 97 | 154 |
| Fat tissues | 90 | 137 |
| Kidney | 105 | 113 |
| Spleen* | 90 | 146 |
| Duodenum | 100 | 136 |
| Liver* | 86 | 226 |
| Heart* | 111 | 259 |
| Brain* | 109 | 163 |

The organs with the mark "*" indicate those where the increase in blood flow was more noticeable than in other organs in the glutamine-administered group.

### [Citation List]

### [Patent Literature]

[PTL 1] U.S. Patent No.2002182162
[PTL 2] Japanese Patent Unexamined Publication (JP Kokai) 2004-262878

### [Non Patent Literature]

[NPL 1] Biomedical Gerontology 2001: 25(2); 83-88
[NPL 2] Folia Pharmacologica Japonica 2002: 119; 7-14
[NPL 3] British Journal of Clinical Pharmacology 1998; 46: 489-497
[NPL 4] Anesthesiology 1994; 80: 1320-13

## Claims

1. A glutamine-containing composition for increasing blood flow, which contains glutamine in an amount ranging from 25 mg/kg body weight to 150 mg/kg body weight per a dose.

2. The composition for increasing blood flow of claim 1, which the amount of glutamine is ranging from 50 mg/kg body weight to 150 mg/kg body weight per a dose.

3. The composition for increasing blood flow of claim 1 or 2, wherein the glutamine is selected from the group consisting of L-glutamine, L-glutamine chlorides, and L-glutamine derivatives that can be metabolized to L-glutamine *in vivo*.

4. The composition for increasing blood flow of any one of claims 1 to 3, wherein, when the composition is packed in a unit, the unit contains 1 to 27 g of glutamine in terms of a free amino acid.

5. The composition for increasing blood flow of any one of claims 1 to 4, which further comprises at least one amino acid selected from the group consisting of pyrrolidone carboxylic acids, pyrrolidone carboxylic acid salts, and pyrrolidone carboxylic acid derivatives.

6. The composition for increasing blood flow of claim 5, wherein the ratio by mass of the glutamine to the amino acid is from 1:4 to 4: 1.

7. A composition for increasing blood flow, which consists of glutamine in an amount ranging from 25 mg/kg body weight to 150 mg/kg body weight per a dose.

8. A pharmaceutical composition for preventing, alleviating or treating dementia, cold constitution, shoulder stiffness, skin muddiness or muscle fatigue, comprising the composition for increasing blood flow of any one of claims 1 to 7.

9. A glutamine-containing food composition for increasing blood flow, which contains glutamine in an amount ranging from 25 mg/kg body weight to 150 mg/kg body weight per a dose.

10. A feed comprising the composition for increasing blood flow of any one of claims 1 to 7.
